(19) Europäisches Patentamt
European Patent Office
Office européen des brevets

(11) Publication number: **0 019 960**
**B1**

(12) **EUROPEAN PATENT SPECIFICATION**

(45) Date of publication of patent specification: **09.11.83**

(51) Int. Cl.³: **C 07 C 11/12**, C 07 C 2/74

(21) Application number: **80200436.6**

(22) Date of filing: **09.05.80**

(54) **Preparation of 3,7-dimethylocta-1,7-diene.**

(30) Priority: **24.05.79 US 42382**

(43) Date of publication of application:
**10.12.80 Bulletin 80/25**

(45) Publication of the grant of the patent:
**09.11.83 Bulletin 83/45**

(84) Designated Contracting States:
**CH DE FR GB LI NL**

(56) References cited:
**EP - A - 0 007 666**
**EP - A - 0 012 472**
**EP - A - 0 012 475**
**US - A - 3 732 328**

**JOURNAL OF ORGANIC CHEMISTRY, vol. 41, No. 21, 15th October 1976, pages 3455—3460 Easton, U.S.A. J. P. NEILAN et al.: "Monoterpene Syntheses via a Palladium Catalyzed Isoprene Dimerization"**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Nozaki, Kenzie**
**1407 Lakecliff Drive**
**Houston, Texas 77077 (US)**

(74) Representative: **Puister, Antonius Tonnis, Mr. et al,**
**P.O. Box 302**
**NL-2501 CH The Hague (NL)**

**EP 0 019 960 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Preparation of 3,7-dimethylocta-1,7-diene

The invention relates to a process for the preparation of 3,7-dimethylocta-1,7-diene by hydrodimerization of isoprene in the presence of a reducing agent based on formic acid, a catalyst comprising palladium complexed with a tertiary organophosphorus ligand and a solvent.

3,7-Dimethylocta-1,7-diene is a chemical intermediate, for example to aroma chemicals.

In the process referred to in the beginning, as described in U.S. patent specification 3,732,328 formic acid is used as the reducing agent. This known process, however, allows 3,7-dimethylocta-1,7-diene with a low selectivity, 3,6-dimethylocta-1,7-diene primarily being formed. The selectivity, expressed as a percentage to a certain compound, is defined as:

$$\frac{a}{b} \times 100,$$

wherein "a" is the amount of isoprene converted to this certain compound and "b" it the total amount of converted isoprene.

Such a process has also been disclosed in J. Org. Chem., Vol. 41, No. 21, 1976, pages 3455—3460, in which process formic acid was used as a reducing agent. However, the selectivity to 3,7-dimethylocta-1,7-diene is mostly under 50% and often the amount of 3,6-dimethylocta-1,7-diene is more than that of 3,7-dimethylocta-1,7-diene; besides the reaction times are rather long.

There has now been found that the use of certain reducing agents allows a high selectivity to 3,7-dimethylocta-1,7-diene, a mixture of dimethylocta-1,7-dienes having greater than fifty mol. per cent of the 3,7-dimethylocta-1,7-diene isomer being formed. Accordingly, the invention provides a process for the preparation of 3,7-dimethylocta-1,7-diene by hydrodimerization of isoprene in the presence of a reducing agent based on formic acid, a catalyst comprising palladium complexed with a tertiary organophosphorus ligand and a solvent, characterized in that the tertiary organo-phosphorus ligand is represented by the formula $(RO)_aPR_b$ wherein "a" is an integer from 0 to 3 and "b" is 3-a, and the R groups, which may be the same or different, represent hydrocarbyl groups with less than 12 carbon atoms and at least one R group represents a sterically hindering group being a branched alkyl group or an aralkyl, alkenyl or cycloalkyl group, the aforementioned groups having 3 to 10 carbon atoms whilst branching occurring in the alkyl groups is at a carbon atom no more than two carbon atoms from the phosphorus or oxygen atom, the solvent is dimethylformamide, N-methylpyrrolidinone or dimethylsulphoxide and lithium formate, sodium formate, ammonium formate or calcium formate or a mixture thereof is used as the reducing agent in the presence of water.

Water must be present in the reaction mixture in greater than trace amounts. Preferred molar amounts of water are at least equal to the molar amounts of formate salt. Typically, the water will range from 1 to 5 mol. of water per mol. of formate salt.

The solvents are dimethylformamide, N-methylpyrrolidinone and dimethyl sulphoxide, which are good solvents for the formate salts in question.

The amount of solvent added should be sufficient to dissolve the palladium complexed with a tertiary organo-phosphorus ligand.

The formate salts are utilized as a source of hydrogen for the hydrodimerization. Sodium formate is preferred.

It is desirable that some formate salt be present during the entire course of the reaction. When operating the process batch-wise, this can be accomplished by adding a stoichiometric amount of formate salt initially, 1 mol. of formate salt for every 2 moles of isoprene, or by continuously or periodically adding additional amounts of formate salt.

The catalyst used in the process of this invention is palladium or a palladium compound complexed with a tertiary organo-phosphorus ligand. The palladium may be in any of its possible valence states, e.g., 0, +2, etc. with the zero valence preferred. Suitable palladium compounds include the palladium carboxylates, particularly palladium carboxylates derived from alkanoic acids containing up to six carbon atoms per molecule such as palladium acetate (OAC), complexes, such as palladium acetylacetonate (AcAc), bis-benzonitrile palladium(II) and lithium palladous chloride as well as the palladium halides, nitrates and sulphates, such as palladous chloride and palladium nitrate $(Pd(NO_3)_2(OH_2)$ and palladium sulphate. Suitable reduced palladium-phosphine complexes are $Pd(R_3P)_2$ or $Pd(R_3P)_3$. The palladium is present in the reaction mixture in catalytic amounts; preferably from 1 to $10^{-6}$ molar and more preferably from $10^{-1}$ to $10^{-4}$ molar.

The palladium compounds complexed with a tertiary organophoshorus ligand are typically prepared by reacting the tertiary organo-phosphorus ligand with the appropriate palladium compound as, for example, represented by the following equations:

$$2R_3P+(PhCN)_2PdCl_2 \rightarrow (R_3P)_2PdCl_2$$

$$(R_3P)_2PdCl_2+AgCO_3\rightarrow(R_3P)_2PdCO_3$$

$$(R_3P)_2PdO_2+SO_2\rightarrow(R_3P)_2PdSO_4$$

$$(R_3P)_2PdO_2+N_2O_4\rightarrow(R_3P)_2Pd(NO_3)_2$$

where $R_3P$ is a tertiary organo-phosphine or may be made in situ by adding the palladium compound and the phosphine directly to the reactor.

The ligands are represented by the formula:

$$(RO)_aPR_b \quad (I)$$

wherein the R groups which may be the same or different, attached to the phosphorus or oxygen atoms, represent hydrocarbyl groups, "a" is an integer from 0 to 3 and "b" is 3-a and at least one R represents a sterically hindering group as defined hereinbelow. The other R in formula I represent each an aryl, alkyl, aralkyl, alkaryl group or a mixture thereof with less than 20 carbon atoms, preferably less than 12 carbon atoms. Suitable examples are phenyl, p-tolyl, o-tolyl, m-tolyl, m-chlorophenyl, methyl, ethyl, propyl and butyl groups with methyl, ethyl, n-propyl, n-butyl, phenyl and chlorophenyl groups being preferred.

In the tertiary organo-phosphorus ligands at least one R represents a branched alkyl or an aralkyl, alkenyl or cycloalkyl group, each of the groups having from 3 to 10 carbon atoms with branching occurring at a carbon atom no more than two carbon atoms from the phosphorus or oxygen atom. This R provides a steric hindrance to the catalyst complex which enhances selectivity.

Illustrative of this R moiety are, for alkyl, isopropyl, sec.-butyl, tert.-butyl, isobutyl, neopentyl, sec.-pentyl, tert.-pentyl, 2-methylbutyl, sec.-hexyl, tert.-hexyl, 2,2-dimethylpropyl; for aralkyl, alpha-methylbenzyl, alpha,alpha-dimethylbenzyl, alpha-methyl-alpha-ethylbenzyl, phenylethyl, phenylisopropyl, phenyl-tert.-butyl; for alkenyl, allyl, crotyl, methallyl, 1-methylethenyl, 1-methyl-2-propenyl, 2-methyl-2-propenyl, 1,1-dimethyl-2-propenyl, 1-methyl-3-butenyl and, for cycloalkyl, cyclopropyl, cyclobutyl, cyclopentyl, cyclohexyl and cycloheptyl.

Two or more of the ligands of formula I may be used in the same reaction. The mol. ratio of tertiary phosphorus ligand to palladium is suitably at least 1. Preferably, the mol. ratio of ligand to palladium ranges from 1:1 to 20:1 and more preferably from 2:1 to 5:1. The use of the tertiary organo-phosphorus ligands of the invention provides extremely high selectivities to 3,7-dimethylocta-1,7-diene.

Alternatively, the palladium or palladium compound and tertiary organo-phosphorus ligand may be bound onto a cross-linked synthetic resin instead of being dissolved in the reaction medium. Suitable cross-linked synthetic resins include cross-linked polystyrene, poly(alpha-alkyl)acrylates, polycarbonates and polyamides.

The number of repeating units substituted with the tertiary phosphine is not critical. When less than 5% of the repeating units contain a phosphorous substitute, large quantities of the resin must be used to form the bound catalyst. Accordingly, it is desirable to have at least 10% of the repeating units substituted with a tertiary phosphorus. It is preferred, however, that from 20 to 40% of the repeating units contain a phosphorus substituent. The substituent can be introduced into the resin using well-known techniques, such as those described in the Journal of the American Chemical Society, *97* (7) 1749 (1975) and in Ann. N.Y. Academy of Sciences, *239*, 76 (1974). In accordance with those techniques, the palladium compound is complexed with the phosphorus-substituted resin by admixing in a solvent for the palladium compound.

The catalyst may be pretreated to enhance reactivity by contacting it with a reducing agent at a temperature of from 20 to 90°C for from 0.1 to 5 hours. The reducing agent may be gaseous, solid or liquid. Examples of gaseous reducing agents are hydrogen and carbon monoxide. Examples of liquid or solid reducing agents are hydrazine, $NaBH_4$, $NaOCH_3$, $(isopropyl)_3P$, Cu, Na, and Al alkyls. The reduction may be carried out in a separate autoclave or preferably is carried out in the hydrodimerization reactor prior to the introduction of the isoprene. The palladium complexed with a tertiary organo-phosphorus ligand may be dissolved in the solvent used in this invention prior to reduction.

The process can be either continuous or batch. The reaction temperature of the process is not critical, however, it is preferred to maintain the reaction between 0 and 100°C, preferably between 20 and 70°C. The process is conducted under a sufficient pressure to maintain liquid phase conditions at the reaction temperature. Typically the pressure is autogenous.

The invention will now be illustrated by the following Examples.

Examples 1—9

To a 90 millilitre glass-lined autoclave were charged 0.027 mmol. of palladium acetylacetonate, 0.054 mmol. $(isopropyl)_3$ phosphine, 18.5 mmoles of formate, 10 ml of the solvent listed in Table I, 1 ml (55.5 mmol.) of $H_2O$ and 2.5 g (37 mmol.) of isoprene. The stirred reactor was heated to various

temperatures for 2 hours, cooled and the product was analyzed by gas chromatography. The results are shown in Table I.

TABLE I

| Example | Formate source | Solvent[a)] | Reaction temp., °C | Conversion, % | Selectivity, % 3,7 DM | 3,6 DM[b)] | 2,7 DM |
|---|---|---|---|---|---|---|---|
| 1 | Na formate | DMSO | 60 | 76 | 65 | 10 | 25 |
| 2 | Na formate | DMSO | 75 | 89 | 65 | 7 | 28 |
| 3 | Na formate | DMSO (20 ml) | 60 | 41 | 73 | 8 | 14 |
| 4 | Na formate | DMF | 75 | 81 | 68 | 7 | 25 |
| 5 | Na formate | DMF (20 ml) | 60 | 65 | 73 | 8 | 18 |
| 6 | $NH_4$ formate | DMSO | 60 | 80 | 55 | 31 | 14 |
| 7 | $NH_4$ formate | DMF | 60 | 86 | 56 | 33 | 11 |
| 8 | Ca formate | DMF | 60 | 61 | 63 | 5 | 32 |
| 9 | Li formate | DMF | 60 | 78 | 58 | 24 | 8 |

[a)]DMF=dimethyl formamide
DMSO=dimethyl sulphoxide

[b)]3,6 DM=3,6-dimethylocta-1,7-diene
3,7 DM=3,7-dimethylocta-1,7-diene
2,7 DM=2,7-dimethylocta-1,7-diene

Comparative experiments 1—9

To compare the different isomer mixes that are obtained using formic acid, amine salt, and other formates the following experiments were run:

To an 80 millilitre glass-lined autoclave were charged 0.027 mmol. of palladium acetylacetonate, 0.054 mmol. (isopropyl)$_3$phosphine, 18.5 mmol. of formate or formic acid, 10 ml of the solvent listed in Table II, 1 ml (55.5 mmol.) of $H_2O$ (for the formates only) and 2.5 g (37 mmol.) of isoprene. The stirred reaction was heated for 2 hours at various temperatures, cooled and the product was analyzed by gas chromatography. The results are shown in Table II.

TABLE II

| Comparative experiment | Formate source | Solvent[a)] | Reaction temp., °C | Conversion, % | Selectivity, % 3,7 DM | 3,6 DM[b)] | 2,7 DM |
|---|---|---|---|---|---|---|---|
| 1 | Formic acid | DMSO | 60 | 87 | 34 | 61 | 4 |
| 2 | Formic acid | DMF | 60 | 75 | 36 | 60 | 4 |
| 3 | Formic acid $(CH_3CH_2)_3N$ | DMF | 40 | 98 | 38 | 58 | 4 |
| 4 | Rb formate | DMF | 60 | 95 | 45 | 47 | 8 |
| 5 | Cs formate | DMF | 60 | 93 | 50 | 43 | 7 |
| 6 | K formate | DMF | 60 | 87 | 30 | 1 | 69 |
| 7 | K formate | DMSO | 72 | 84 | 39 | 1 | 60 |
| 8 | K formate | DMF | 72 | 81 | 25 | 1 | 74 |
| 9 | K formate | DMF | 85 | 91 | 11 | 1 | 88 |

[a)]DMF=dimethyl formamide
DMSO=dimethyl sulphoxide

[b)]3,6 DM=3,6-dimethylocta-1,7-diene
3,7 DM=3,7-dimethylocta-1,7-diene
2,7 DM=2,7-dimethylocta-1,7-diene

## Claims

1. A process for the preparation of 3,7-dimethylocta-1,7-diene by hydrodimerization of isoprene in the presence of a reducing agent based on formic acid, a catalyst comprising palladium complexed with a tertiary organophosphorus ligand and a solvent, characterized in that the tertiary organophosphorus ligand is represented by the formula

$$(RO)_aPR_b$$

wherein "a" is an integer from 0 to 3 and "b" is 3-a, and the R groups, which may be the same or different, represent hydrocarbyl groups with less than 12 carbon atoms and at least one R group represents a sterically hindering group being a branched alkyl group or an aralkyl, alkenyl or cycloalkyl group, the aforementioned groups having 3 to 10 carbon atoms whilst branching occurring in the alkyl groups is at a carbon atom no more than two carbon atoms from the phosphorus or oxygen atom, the solvent is dimethylformamide, N-methylpyrrolidinone or dimethylsulphoxide, and lithium formate, sodium formate, ammonium formate or calcium formate or a mixture thereof is used as the reducing agent in the presence of water.

2. A process as claimed in claim 1, characterized in that at least 1 mol of water per mol of formate is present.

3. A process as claimed in claim 2, characterized in that from 1 to 5 mol of water per mol of formate is present.

**Patentansprüche**

1. Ein Verfahren zur Herstellung von 3,7-Dimethylocta-1,7-dien durch Hydrodimerisierung von Isopren in Anwesenheit eines Reduktionsmittels auf der Grundlage von Ameisensäure, eines Katalysators enthaltend Palladium komplexiert mit einem tertiären Organophosphorliganden, und eines Lösungsmittels, dadurch gekennzeichnet, daß der tertiäre Organophosphorligand durch die Formel

$$(RO)_aPR_b$$

wiedergegeben wird, in welcher "a" eine ganze Zahl von 0 bis 3 und "b" 3-a ist und die Gruppen R, welche gleich oder verschieden sein können, Kohlenwasserstoffgruppen mit weniger als 12 Kohlenstoffatomen bedeuten und mindestens eine Gruppe R eine sterisch gehinderte Gruppe in Form einer verzweigten Alkylgruppe oder einer Aralkyl-, Alkenyl- oder Cycloalkylgruppe darstellt, wobei die vorstehen genannten Gruppen 3 bis 10 Kohlenstoffatome aufweisen, wobei die in den Alkylgruppen auftretende Verzweigung an einem Kohlenstoffatom vorliegt, welches nicht mehr als 2 Kohlenstoffatome von dem Phosphor- oder Sauerstoffatom entfernt ist, das Lösungsmittel Dimethylformamid, N-Methylpyrrolidinon oder Dimethylsulfoxid ist, und Lithiumformat, Natriumformat, Ammoniumformat oder Calciumformat oder eine Mischung davon in Anwesenheit von Wasser als Reduktionsmittel verwendet wird.

2. Ein Verfahren wie in Anspruch 1 beansprucht, dadurch gekennzeichnet, daß mindestens 1 Mol Wasser je Mol Format anwesend ist.

3. Ein Verfahren wie in Anspruch 2 beansprucht, dadurch gekennzeichnet, daß von 1 bis 5 Mol Wasser je Mol Format anwesend sind.

**Revendications**

1. Un procédé pour la préparation de 3,7-diméthylocta-1,7-diène par hydrodimérisation d'isoprène en présence d'un agent réducteur à base d'acide formique, d'un catalyseur comprenant du palladium complexé avec un ligand organo-phosphore tertiaire et d'un solvant, caractérisé en ce que le ligand organo-phosphore tertiaire est représenté par la formule

$$(RO)_aPR_b$$

dans laquelle "a" est un nombre entier de 0 à 3 et "b" est 3-a, et les groupes R, qui peuvent être identiques ou différents, représentent des groupes hydrocarbyle de moins de 12 atomes de carbone et au moins un groupe R représente un groupe causant un empêchement stérique qui est un groupe alcoyle ramifié ou un groupe aralcoyle, alcényle ou cycloalcoyle, les groupes mentionnés ci-dessus ayant 3 à 10 atomes de carbone tandis que la remification présente dans les groupes alcoyle se trouve à un atome de carbone situé à pas plus de deux atomes de carbone de l'atome de phosphore ou d'oxygène, le solvant est du diméthylformamide, de la N-méthylpyrrolidinone ou du diméthylsulfoxyde et du formiate de lithium, du formiate de sodium, du formiate d'ammonium ou du formiate de calcium ou un mélange de ces composés est utilisé comme agent réducteur en présence d'eau.

2. Un procédé selon la revendication 1, caractérisé en ce qu'au moins 1 mole d'eau est présente par mole de formiate.

3. Un procédé selon la revendication 2, caractérisé en ce que 1 à 5 moles d'eau sont présentes par mole de formiate.